# EUROPEAN PATENT APPLICATION

(11) **EP 4 600 166 A1**
(43) Date of publication of application: **13.08.2025**
(21) Application number: 24215845.9
(22) Date of filing: 27.11.2024
(51) Int. Cl.: B65B 55/08, B67B 3/00, B65B 61/18, A61L 2/10

(54) **STERILIZING OF OPENING DEVICES OF PACKAGES**

(30) Priority: 07.02.2024 EP 24156189
(71) Applicant: Tetra Laval Holdings & Finance S.A., 1009 Pully (CH)
(72) Inventor: Carmo, Rui Manuel, 22186 Lund (SE); Floderus, Anders, 22186 Lund (SE)
(74) Representative: Tetra Pak Patent Attorneys

(57) **Abstract**

The present invention relates to an opening device sterilizer for sterilizing opening devices having an inner top surface comprising a circular protruding feature. The opening device sterilizer comprising a sterilizing unit arranged in a conveyor line configured to transport opening devices along a conveyance path. The sterilizing unit comprises a first set of UV-LEDs configured to, during operation, emit UV-light towards the inner top surface of the opening devices. The first set of UV-LEDs are arranged in a first pattern forming one or more rings of UV-LEDs, wherein a diameter of each ring of UV-LEDs in the first pattern corresponds to a diameter of the circular protruding features of the opening devices. Also a system comprising the opening device sterilizer and a method for sterilizing opening devices are presented.

## Description

### Technical field

The present invention relates to the field of packaging technology. More particularly, it is related to methods and apparatuses for pre-sterilizing an opening device before it is placed in a pre-cut hole of a package and attaching thereto.

### Background of the invention

Within the food industry today, the food package technology plays an important part. The food package has several important functions. Apart from branding of the product and presenting the customers with information, the food package also has an important role of ensuring food safety. The packaging materials used in the food package can be designed to provide strength and stability, so that the packages are not damaged during transportation. Furthermore, the packaging materials can form a protective environment for the food product so that it is protected from for example bacteria, germs, oxygen and sun light, thus prolonging shelf life. However, the packaging material is not the only thing that is important in the package. An interior of the package need to be as sterile as possible before filling the package with food. This in order to increase shelf life of the food in the package.

In many food packages of today an opening device is present. Such opening devices typically comprises a spout provided with an attachment flange and a cap (e.g. a screw cap) arranged to interact with the spout. During production of a package the opening device is typically applied to the package using an opening device applicator by placing the opening device in a pre-cut hole of the package and attaching the attachment flange of the opening device onto an attachment section on an inside of the package. However, in order for the interior of the package to be as sterile as possible the interior of the opening device can be sterilized before attaching it to the package.

However, performing such sterilization of opening devices before attaching them to packages comes with a challenge. Especially since an interior surface of the opening device typically comprises various protruding features, such as a circular lip for sealing the cap to the spout, a sealing membrane, knifes for breaking a seal in the opening device, etc.

### Summary of the invention

The herein disclosed technology seeks to at least partly mitigate, alleviate or eliminate one or more of the above-mentioned deficiencies and disadvantages in the prior art. In particular, it is an object to provide pre-sterilization of opening devices to be attached to food packages.

The inventors of the present inventive concept have realized a new and improved way of sterilizing opening devices before they are attached to packages. The present inventive concept also present a new and improved way of sterilizing packages comprising opening devices.

Various aspects and embodiments of the disclosed invention are defined below and in the accompanying independent and dependent claims.

According to a first aspect, there is provided an opening device sterilizer for sterilizing opening devices having an inner top surface comprising a circular protruding feature. The opening device sterilizer comprising a sterilizing unit arranged in a conveyor line configured to transport opening devices along a conveyance path. The sterilizing unit comprises a first set of UV-LEDs configured to, during operation, emit UV-light towards the inner top surface of the opening devices. The first set of UV-LEDs are arranged in a first pattern forming one or more rings of UV-LEDs, wherein a diameter of each ring of UV-LEDs in the first pattern is the same as is within a range of 10 to 50 mm, such that the diameter of each ring of UV-LEDs in the first pattern corresponds to a diameter of the circular protruding features of the opening devices.

The sterilizing unit may comprise a second set of UV-LEDs configured to, during operation, emit UV-light towards the inner top surface of the opening devices. The second set of UV-LEDs may be arranged in a second pattern forming one or more rings of UV-LEDs. A diameter of each ring of UV-LEDs in the second pattern is the same and is different from the diameter of the one or more rings of UV-LEDs in the first pattern. The diameter of the second pattern may be smaller than the diameter of the first pattern.

The sterilizing unit may comprise a plurality of sub-stations. Each sub-station comprising a first set of UV-LEDs arranged in a ring of LEDs. The ring of LEDs having the diameter of the first pattern.

Each sub-station may further comprise a second set of UV-LEDs arranged in a ring of LEDs having the diameter of the second pattern.

An angular pitch of the UV-LEDs in a ring of UV-LEDs in a upstream sub-station may be rotated in relation to the UV-LEDs in a ring of UV-LEDs in a downstream sub-station.

The opening device sterilizer may further comprise a tunnel structure. The tunnel structure being configured to house the sterilizing unit. The tunnel structure being configured to prevent UV-light from escaping the sterilizing unit. The tunnel structure comprising an inlet opening for an inlet of opening devices and an outlet opening for an outlet of opening devices.

The opening device sterilizer may further comprise the conveyor line.

The conveyor line may be arranged for intermittent transport of the opening devices.

The opening device sterilizer may further comprise an overlap adjusting mechanism configured for adjustment of the sterilizing unit with respect to the circular protruding features of the opening devices. Especially, the adjusting mechanism is configured for adjustment of the sterilizing unit such that the one or more rings of UV-LEDs in the first pattern overlap with the circular protruding features of the opening devices arranged in the conveyor line upon the opening devices being intermittently transported.

According to a second aspect a system for sterilizing packages for food is provided. The system comprising: an opening device sterilizer according to the first aspect for sterilizing surfaces of opening devices facing an interior of the package, the opening devices to be attached to the packages; an opening device applicator configured to place a respective opening device , being sterilized by the opening device sterilizer, in a respective pre-cut hole of a respective package and attach an attachment flange of the respective opening device onto an attachment section on an inside of the respective package; and a sterilizing module configured to sterilize an interior of the respective package as well as a bottom surface of the attachment flange of the respective opening device. The sterilizing module is configured to sterilize an interior of the respective package as well as a bottom surface of the attachment flange of the respective opening device by emitting UV-light from a plurality of UV-LEDs into the interior of the package. Alternatively, or in combination, sterilizing module is configured to sterilize an interior of the respective package as well as a bottom surface of the attachment flange of the respective opening device by exposing the interior of the package for peroxide using a peroxide exposing unit.

The system may further comprise a conveyor line configured to transport opening devices along a conveyance path. The sterilizing unit may be arranged along the conveyance path, the conveyor line extending from a source of opening devices to the opening device applicator. The conveyor line may comprise guiding members extending in a transport direction of the opening devices along the conveyance path. The opening devices may be placed in a queue with overlapping attachment flanges in the conveyor line.

The sterilizing module may comprise a plurality of UV-LEDs configured to emit UV-light into the interior of the package.

The above mentioned features of the opening device sterilizer, when applicable, apply to this second aspect as well. In order to avoid undue repetition, reference is made to the above.

According to a third aspect a method for sterilizing opening devices, each opening device comprising a spout provided with an attachment flange and a cap arranged to interact with the spout, wherein the opening device has an inner top surface comprising a circular protruding feature, is provided. The method comprising: sterilizing the inner top surface comprising the circular protruding feature of the opening devices during transport of the opening devices to an opening device applicator configured to apply the opening devices to packages configured to contain liquid food, wherein the sterilizing is performed using UV-light emitted by a plurality of UV-LEDs ; applying the opening device to the package at the opening device applicator by placing the opening device in a pre-cut hole of the package and attaching the attachment flange onto an attachment section on an inside of the package; and sterilizing an interior of the package as well as a bottom surface of the attachment flange of the opening device applied onto the package using a sterilizing module. The plurality of UV-LEDs are arranged as at least two rings of UV-LEDs. The rings of UV-LEDs having different diameters. The sterilizing the inner top surface may comprising the circular protruding feature of the opening devices may comprise aligning a diameter of one of the two rings of UV-LEDs with a diameter of the protruding feature.

Sterilizing an interior of the package as well as a bottom surface of the attachment flange of the opening device applied onto the package may comprise UV-light treatment by emitting UV-light from a plurality of UV-LEDs of the sterilizing module into the interior of the package.

Sterilizing an interior of the package as well as a bottom surface of the attachment flange of the opening device applied onto the package comprises peroxide treatment by exposing the interior of the package for peroxide using a peroxide exposing unit of the sterilizing module.

A further scope of applicability of the present disclosure will become apparent from the detailed description given below. However, it should be understood that the detailed description and specific examples, while indicating preferred variants of the present inventive concept, are given by way of illustration only, since various changes and modifications within the scope of the inventive concept will become apparent to those skilled in the art from this detailed description.

### Brief description of the drawings

The above and other aspects of the present inventive concept will now be described in more detail, with reference to appended drawings showing variants of the present inventive concept. The figures should not be considered limiting the invention to the specific variant; instead, they are used for explaining and understanding the inventive concept.

As illustrated in the figures, the sizes of layers and regions are exaggerated for illustrative purposes and, thus, are provided to illustrate the general structures of variants of the present inventive concept. Like reference numerals refer to like elements throughout.
Figs 1a-1c illustrates different examples of opening devices to be attached to a pre-cut hole of a package, especially a cartoon based food package.
Figs 2a-d schematically illustrates an opening device sterilizer for sterilizing opening devices of Fig. 1.
Fig. 3 schematically illustrates system for sterilizing opening devices of Fig. 1.
Fig. 4 is a flow chart illustrating a method for sterilizing opening devices of Fig. 1.

### Detailed description

The present inventive concept will now be described more fully hereinafter with reference to the accompanying drawings, in which currently preferred variants of the inventive concept are shown. This inventive concept may, however, be implemented in many different forms and should not be construed as limited to the variants set forth herein; rather, these variants are provided for thoroughness and completeness, and fully convey the scope of the present inventive concept to the skilled person.

It will also be appreciated that when the present disclosure is described in terms of a method, it may also be embodied in an apparatus or device comprising one or more processors, one or more memories coupled to the one or more processors, where computer code is loaded to implement the method. For example, the one or more memories may store one or more computer programs that perform the steps, services and functions disclosed herein when executed by the one or more processors.

It is also to be understood that the terminology used herein is for the purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. The term "obtaining" is herein to be interpreted broadly and encompasses receiving, retrieving, collecting, acquiring, and so forth.

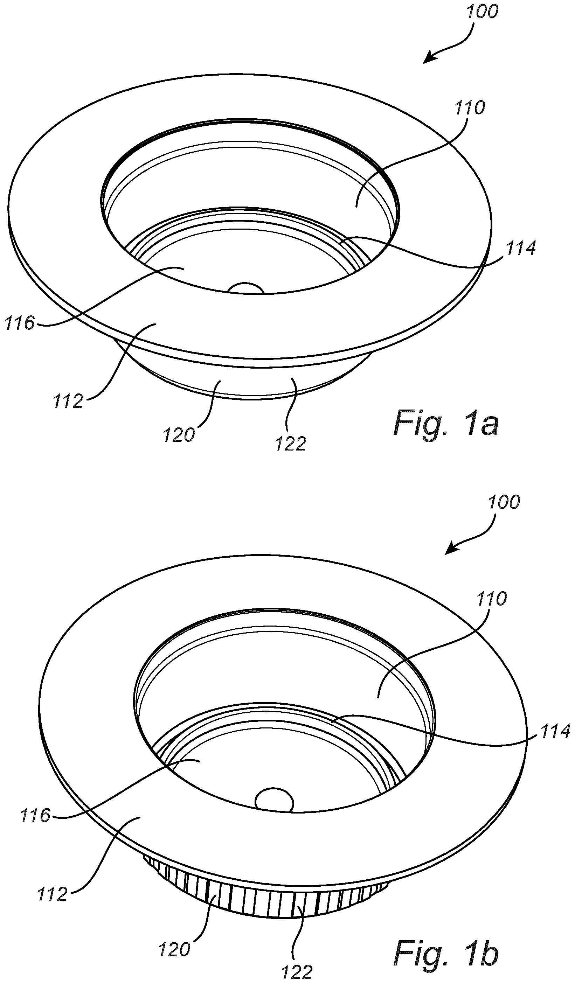

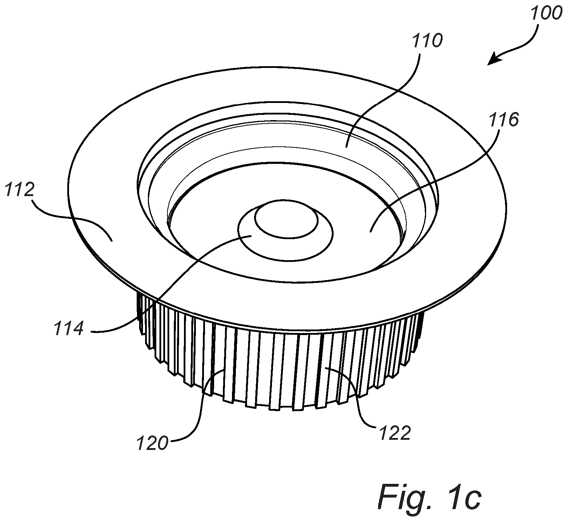

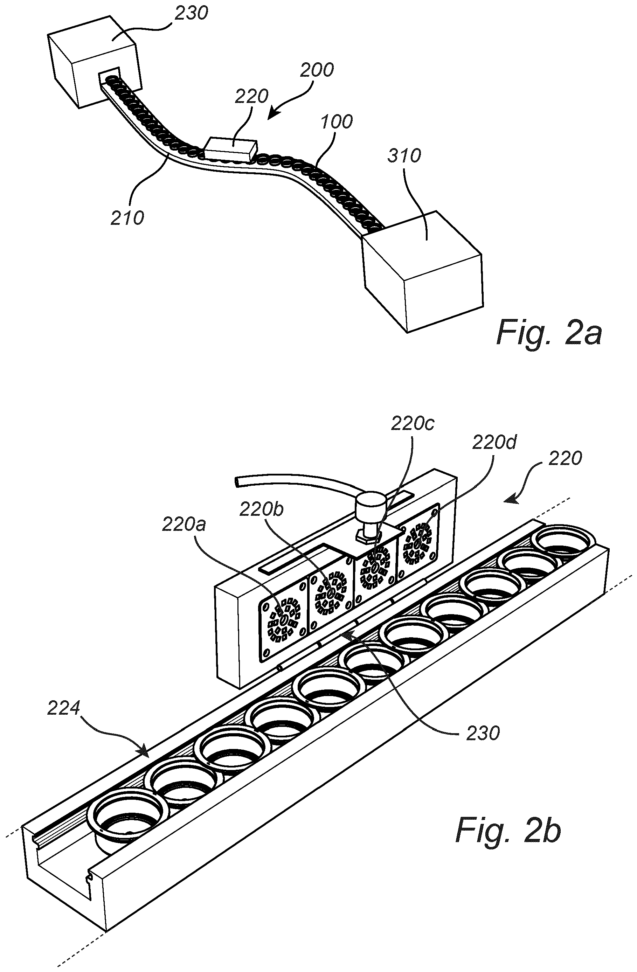

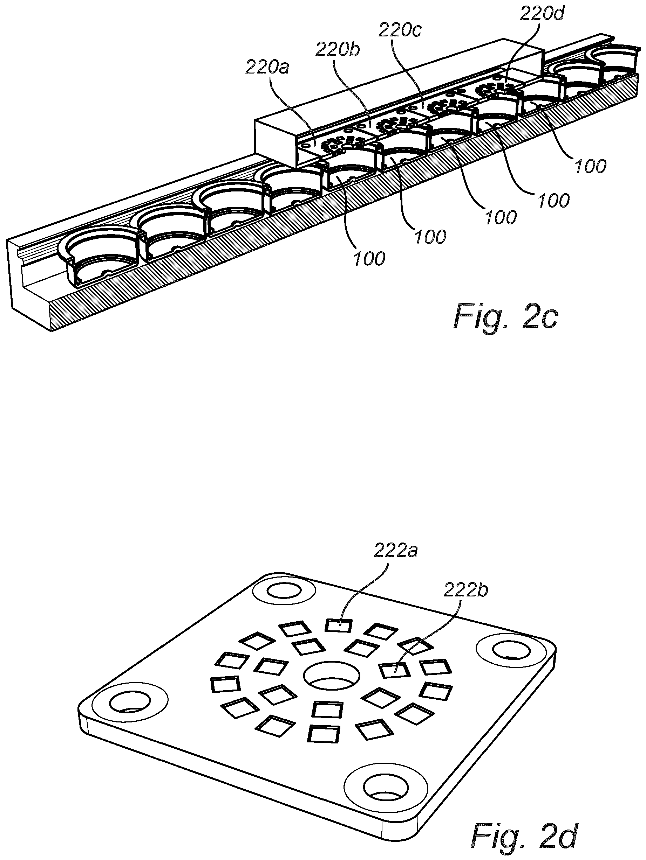

An opening device sterilizer 200 for sterilizing opening devices 100 to be attached to food packages, as well as a system 300 for sterilizing opening devices 100 and a method 400 for sterilizing opening devices 100 will now be described with reference to Fig. 2 to Fig. 4. The opening devices 100 comprising a spout 110 provided with an attachment flange 112 and a cap 120 (e.g. a screw cap) arranged to interact with the spout 110. During production of a package the opening device 100 is typically attached to the package using an opening device applicator 310 by placing the opening device 100 in a pre-cut hole of the package and attaching the attachment flange 112 of the opening device 100 onto an attachment section on an inside of the package. Examples of opening devices 100 are illustrated in connection with Figs 1a-c. Common for the different types of opening devices 100 illustrated in connection with Figs 1a-c is that they all comprises a circular protruding feature 114 on an inner top surface 116 of the opening device 100. For the opening devices 100 illustrated in connection with Figs 1a and 1b the circular protruding feature 114 is a lip configured to provide a seal between the spout 110 and the cap 120. For the opening device 100 illustrated in connection with Fig. 1c the circular protruding feature 114 is a portion of a membrane to be teared off once the opening device 100 is opened for the first time by a consumer of the food in the package. Opening devices 100 comprising other types of circular protruding features 114 may also be considered.

Figs. 2a-d schematically illustrates an opening device sterilizer 200 for sterilizing opening devices 100 to be attached to food packages. Examples of opening devices 100 that may be sterilized in the opening device sterilizer 200 are illustrated in connection with Figs 1a-1c. The opening device sterilizer 200 comprises a sterilizing unit 220 arranged in a conveyor line 210 configured to transport opening devices 100 along a conveyance path.

The conveyor line 210 may be seen as a part of the opening device sterilizer 200. The conveyance path of conveyor line 210 extends from a source 230 of opening devices 100 to the opening device applicator 310 configured to attach a respective opening device 100 to a respective package. Somewhere along the conveyance path the sterilizing unit 220 is arranged, preferably along a straight portion of the conveyance path. The conveyor line 210 comprises guiding members that extend in a transport direction of the opening devices 100 along the conveyance path. The conveyor line 210 is typically arranged for intermittent transport of the opening devices 100. The intermittent transport is typically controlled by the opening device applicator 310. A frequency of the intermittent transport depend on a number of opening device 100 the opening device applicator 310 is configured to attach per unit time. According to one example the opening device applicator 310 is configured to attach 7000 opening devices per hour. According to this example, a dwell time for the opening devices in each step of the intermittent transport is approximately 0.5 seconds.

The sterilizing unit 220 is illustrated in more detail in Figs 2b and 2c. The sterilizing unit 220 is arranged in the conveyor line 210. The sterilizing unit 220 comprises a first set of UV-LEDs 222a. The UV-LEDs are arranged to emit ultra violet, UV, light. Preferably, the UV-LEDs of the first set of UV-LEDs 222a are arranged to emit UV-light having a peak wavelength in the range of 260-275 nm, more preferably in the range of 265-275 nm. Two examples of suitable UV-LEDs that can be used are OSRAM OSLON^{®} UV 6060, SU CZHEF1.VC and OSRAM OSLON^{®} UV 3535, SU CULEP1.VC. As understood by the skilled person, other UV-LEDs may of course be used. Further, as also understood by the skilled person, in the future UV-LEDs with higher power than available today will be developed. The higher power of the UV-LEDs the better the sterilization will be. The first set of UV-LEDs 222a may all be of a same type having a same peak wavelength. Having, all UV-LEDs in the first set of UV-LEDs 222a with the same peak wavelength will provide as high exposure as possible at that peak wavelength. Alternatively, the first set of UV-LEDs 222a may comprise two or more different types of UV-LEDs having different peak wavelengths. Having different peak wavelengths may be beneficial since different microorganism may have a different sensibility for different wavelengths. During operation of the sterilizing unit 220, the first set of UV-LEDs 222a are configured to emit UV-light towards the inner top surface 116 of the opening devices 100. Hence, an interior of the opening devices 100 are exposed for UV-light during operation of the sterilizing unit 220. As being illustrated in more detail in Figs 2b and 2d, the first set of UV-LEDs 222a are arranged in a first pattern forming one or more rings of LEDs. A diameter of a ring of LEDs in the first pattern correspond to a diameter of the circular protruding feature 114 of the opening devices 100. In case the first pattern comprises a plurality of rings of LEDs these rings of LEDs have a same diameter. Each ring of the plurality of rings of LEDs in the first pattern belonging to a separate one of the substations 220a, 220b, 220c, 220d of the sterilizing unit 220, see Figs 2b and 2c and the discussion below on substations 220a, 220b, 220c, 220d. By the wording "correspond" it is herein meant that the diameter of the ring of UV-LEDs and the diameter of the circular protruding feature 114 of the opening devices 100 are within 90% to 110% with each other. Hence, the two diameters may be the same. Alternatively, there may be a ±10% difference in the diameters. Such difference may be present due to emission angle of the UV-light emitted by the UV-LEDs. The diameter of the one or more rings of LEDs in the first pattern is preferably within a range of 10-50 mm. According to one specific embodiment, the diameter of the one or more rings of LEDs in the first pattern is 22 mm.

The sterilizing unit 220 may further comprises a second set of UV-LEDs 222b, as being illustrated in Figs 2b and 2d. The UV-LEDs of the second set of LEDs 222b may also be arranged to emit UV-light having a peak wavelength in the range of 260-275 nm, more preferably in the range of 265-275 nm. The UV-LEDs of the second set of LEDs 222b may have a same peak wavelength as the UV-LEDs of the first set of LEDs 222a. Having, a second set of LEDs with the same peak wavelength will increase the power at that wavelength. Alternatively, the UV-LEDs of the second set of LEDs 222b may have a different peak wavelength as the UV-LEDs of the first set of LEDs 222a. Having different peak wavelengths may be beneficial since different microorganism may have a different sensibility for different wavelengths. During operation of the sterilizing unit 220, the second set of UV-LEDs 222b are configured to emit UV-light towards the inner top surface 116 of the opening devices 100. The second set of UV-LEDs 222b are arranged in a second pattern forming one or more rings of LEDs. In case the second pattern comprises a plurality of rings of LEDs these rings of LEDs have a same diameter. Each ring of the plurality of rings of LEDs in the second pattern belonging to a separate one of the substations 220a, 220b, 220c, 220d of the sterilizing unit 220, see Figs 2b and 2c and the discussion below on substations 220a, 220b, 220c, 220d. A diameter of a ring of LEDs in the second pattern is different than the diameter of the ring of the LEDs in the first pattern. Preferably, a ring of LEDs of the second pattern is arranged within a ring of LEDs of the first pattern. The diameter of the one or more rings of LEDs in the second pattern is preferably within a range of 5-40 mm. According to one specific embodiment, the diameter of the one or more rings of LEDs in the second pattern is 12 mm. Having two sets of UV-LEDs arranged in ring shapes of different diameters may allow for the sterilizing unit 220 to be operated to sterilize different kinds of opening devices having circular protruding features 114 of different diameter. Alternatively or in combination, having two sets of UV-LEDs arranged in ring shapes of different diameters allow for an increased sterilization to be obtained.

The sterilizing unit 220 may comprise a plurality of sub-stations 220a, 220b, 220c, 220d. During operation of the sterilizing unit 220 each opening device 100 is irradiated with UV-light in each sub-station 220a, 220b. Accordingly, the number of sub-station will affect the dwell time in the sterilizing unit 220. According to the above discussed example, in which the dwell time for each step in the intermittent transport of the opening devices 100 in the conveyor line 210 was 0.5 seconds, the total dwell time in a sterilizing unit 220 having four sub-stations will then be 4 seconds. The desired dwell time in the sterilizing unit 220 depend on which species of microorganism is/are targeted for in the sterilization. For example, Bacillus Atrophaeus is easier to kill off using UV-light than Aspergillus Brasiliensis. Hence, if Aspergillus Brasiliensis is targeted for the sterilization longer dwell times are needed. If additional sterilization of the opening devices 100 is desired the opening devices 100 may additionally also be exposed for peroxide, for example hydrogen peroxide. Hence, the opening device sterilizer 200 may also comprise a peroxide exposing unit configure to expose the opening devices 100 for a peroxide treatment.

Each sub-station 220a, 220b, 220c, 220d may comprise part of the UV-LEDs of the first set of UV-LEDs 222a. Hence, the first pattern comprises a plurality of rings of LEDs, each such ring of LEDs being arranged at a respective one of the plurality of sub-stations 220a, 220b, 220c, 220d. Further, each sub-station 220a, 220b, 220c, 220d may comprise part of the UV-LEDs of the second set of UV-LEDs 222b. Hence, the second pattern comprises a plurality of rings of LEDs, each such ring of LEDs being arranged at a respective one of the plurality of sub-stations 220a, 220b, 220c, 220d.

Further, an angular pitch of the UV-LEDs in a ring of UV-LEDs in a upstream sub-station may be rotated in relation to the UV-LEDs in a ring of UV-LEDs in a downstream sub-station.

The degree of sterilization in the sterilizing unit 220 depend on the power of the UV-LEDs, the number of UV-LEDs, the peak wavelength of the UV-light emitted by the UV-LEDs and the dwell time in the sterilizing unit 220. The dwell time in the sterilizing unit 220 may be altered by one or more of a number of sub-stations 220a, 220b, 220c, 220d and a frequency of the intermittent transport of opening devices in the conveyor line 210.

The opening device sterilizer 200 may further comprise a tunnel structure 224. The tunnel structure 224 may e.g. be in the form of a housing enclosing the sterilizing unit 220. Hence, the tunnel structure 224 is configured to house the sterilizing unit 220. The tunnel structure 224 is further configured to prevent UV-light from escaping the sterilizing unit 220. Accordingly, the tunnel structure 224 is to be made from a material being opaque to UV-light. The tunnel structure 224 comprising an inlet opening for an inlet of opening devices 100 and an outlet opening for an outlet of opening devices 100.

The opening device sterilizer 200 may further comprise an overlap adjusting mechanism 230. The overlap adjusting mechanism 230 is configured for adjustment of the sterilizing unit 220 such that the first pattern of the first set of UV-LEDs 222a overlap with the circular protruding features 114 of the opening devices 100 arranged in the conveyor line 210 during operation. Hence, the overlap adjusting mechanism 230 may be used to adjust so that the one or more rings of UV-LEDs in the first pattern overlap with the circular protruding features 114 of the opening devices 100 upon the opening devices 100 being intermittently transported in the conveyor line 210.

The opening device sterilizer 200 may further comprises a gate stopper (not shown) configured to be switched between a closed state in which a transport of the opening devices 100 is hindered and an open state in which the transport of the opening devices 100 is allowed to continue moving downstream the conveyance path. Such gate stopper may be used at start and stop of operation of the opening device sterilizer 200.

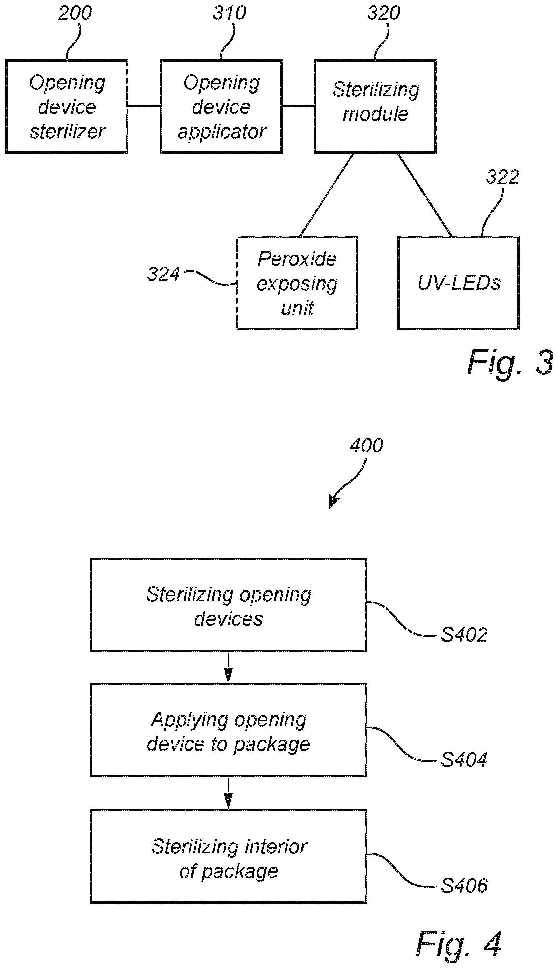

Fig. 3 schematically illustrates system 300 for sterilizing packages, typically carton based food packages. The packages comprising opening devices 100. Examples of opening devices 100 are illustrated in connection with Figs 1a-1c. The system 300 comprises an opening device sterilizer 200 as described above for sterilizing surfaces of the opening devices 100 facing the interior of the packages. The opening devices 100 are typically placed in a queue with overlapping attachment flanges 112 in the conveyor line 210. By using gravity or in any other way pushing the opening devices 100 in a line typically results in that there are overlapping attachment flanges 112. Hence, it cannot be safeguarded that the opening devices 100 are fully sterilized in the opening device sterilizer 200. The system 300 further comprises an opening device applicator 310 configured to place the opening device 100, being sterilized by the opening device sterilizer 200, in a pre-cut hole of a package and attach the attachment flange112 of the opening device 100 onto an attachment section on an inside of the package.

The system 300 further comprises a sterilizing module 320 configured to sterilize an interior of the package as well as a bottom surface of the attachment flange 112 of the opening device 100. The sterilizing module 320 may comprise a plurality of UV-LEDs 322. The UV-LEDs 322 are arranged to emit ultra violet, UV, light. Preferably, the UV-LEDs 322 are arranged to emit UV-light having a peak wavelength in the range of 260-275 nm, more preferably in the range of 265-275 nm. Two examples of suitable UV-LEDs that can be used are OSRAM OSLON^{®} UV 6060, SU CZHEF1.VC and OSRAM OSLON^{®} UV 3535, SU CULEP1.VC. The sterilizing module 320 may further comprise a peroxide exposing unit 324 configure to expose the interior of the package for a peroxide treatment. The peroxide exposing unit 324 may e.g. use hydrogen peroxide.

Fig. 4 is a flow chart illustrating the steps of a method 400 for sterilizing opening devices 100. Examples of opening devices 100 that may be sterilized by the method are illustrated in connection with Figs 1a-1c. Below, the different steps are described in more detail. Even though illustrated in a specific order, the steps of the method 400 may be performed in any suitable order, in parallel, as well as multiple times.

Sterilizing S402 the inner top surface 116 comprising the circular protruding feature 114 of the opening devices 100 during transport of the opening devices 100 to an opening device applicator 310 configured to apply the opening devices 100 to packages configured to contain liquid food. The sterilizing S402 is performed using UV-light emitted by a plurality of UV-LEDs. The plurality of UV-LEDs are arranged as one or more rings of LED:s. The plurality of UV-LEDs may be arranged as at least two rings of UV-LEDs. The at least two rings of UV-LEDs having different diameters. The sterilizing S402 the inner top surface may comprising the circular protruding feature 114 of the opening devices 100 may comprise aligning a diameter of one of the one or more rings of UV-LEDs with a diameter of the protruding feature 114.

Applying S404 the opening device 100 to the package at the opening device applicator 310 by placing the opening device 100 in a pre-cut hole of the package and attaching the attachment flange 112 onto an attachment section on an inside of the package.

Sterilizing S406 an interior of the package as well as a bottom surface of the attachment flange 112 of the opening device 100 applied onto the package using an sterilizing module 320. Sterilizing S406 an interior of the package as well as a bottom surface of the attachment flange 112 of the opening device 100 applied onto the package may comprise UV-light treatment by emitting UV-light from a plurality of UV-LEDs 322 of the sterilizing module 320 into the interior of the package. Alternatively, or in combination, sterilizing S406 an interior of the package as well as a bottom surface of the attachment flange 112 of the opening device(100 applied onto the package may comprise peroxide treatment by exposing the interior of the package for peroxide using a peroxide exposing unit 324 of the sterilizing module 320.

Additionally, variations to the disclosed variants can be understood and effected by the skilled person in practicing the claimed invention, from a study of the drawings, the disclosure, and the appended claims.

## Claims

1. An opening device sterilizer (200) for sterilizing opening devices (100) having an inner top surface (116) comprising a circular protruding feature (114), the opening device sterilizer (200) comprising:
a sterilizing unit (220) arranged in a conveyor line (210) configured to transport opening devices (100) along a conveyance path,
wherein the sterilizing unit (220) comprises a first set of UV-LEDs (222a) configured to, during operation, emit UV-light towards the inner top surface (116) of the opening devices (100),
wherein the first set of UV-LEDs (222a) are arranged in a first pattern forming one or more rings of UV-LEDs, wherein a diameter of each ring of UV-LEDs in the first pattern is the same as is within a range of 10 to 50 mm, such that the diameter of each ring of UV-LEDs in the first pattern corresponds to a diameter of the circular protruding features (114) of the opening devices (100), and
wherein the sterilizing unit (220) further comprises a second set of UV-LEDs (222b) configured to, during operation, emit UV-light towards the inner top surface (116) of the opening devices (100), wherein the second set of UV-LEDs (222b) are arranged in a second pattern forming one or more rings of LEDs, wherein a diameter of each ring of UV-LEDs in the second pattern is the same and is different from the diameter of the one or more rings of UV-LEDs in the first pattern.

2. The opening device sterilizer (200) according to claim 1, wherein the sterilizing unit (220) comprises a plurality of sub-stations (220a, 220b, 220c, 220d), each sub-station (220a, 220b, 220c, 220d) comprising a first set of UV-LEDs (222a) arranged in a ring of LEDs having the diameter of the first pattern.

3. The opening device sterilizer according to claim 1 and 2, wherein each sub-station (220a, 220b, 220c, 220d) comprises a second set of UV-LEDs (222b) arranged in a ring of LEDs having the diameter of the second pattern.

4. The opening device sterilizer according to claim 2 or 3, wherein an angular pitch of the UV-LEDs in a ring of UV-LEDs in a upstream sub-station is rotated in relation to the UV-LEDs in a ring of UV-LEDs in a downstream sub-station.

5. The opening device sterilizer (200) according to any one of claims 1-4, further comprising a tunnel structure (224) configured to house the sterilizing unit (220) and to prevent UV-light from escaping the sterilizing unit (220), the tunnel structure (224) comprising an inlet opening for an inlet of opening devices (100) and an outlet opening for an outlet of opening devices (100).

6. The opening device sterilizer according to any one of claims 1-5, further comprising the conveyor line (210).

7. The opening device sterilizer according to claim 6, wherein the conveyor line (210) is arranged for intermittent transport of the opening devices (100).

8. The opening device sterilizer (200) according to claim 7, further comprising an overlap adjusting mechanism (230) configured for adjustment of the sterilizing unit (220) with respect to the circular protruding features (114) of the opening devices (100) such that the one or more rings of UV-LEDs (222a) in the first pattern overlap with the circular protruding features (114) of the opening devices (100) arranged in the conveyor line (210) upon the opening devices (100) being intermittently transported.

9. A system (300) for sterilizing packages for food, the system (300) comprising:
an opening device sterilizer (200) according to any one of claims 1-8 for sterilizing surfaces of opening devices (100) facing an interior of the package, the opening devices (100) to be attached to the packages;
an opening device applicator (310) configured to place a respective opening device (100), being sterilized by the opening device sterilizer (200), in a respective pre-cut hole of a respective package and attach an attachment flange (112) of the respective opening device (100) onto an attachment section on an inside of the respective package; and
a sterilizing module (320) configured to sterilize an interior of the respective package as well as a bottom surface of the attachment flange (112) of the respective opening device (100) by emitting UV-light from a plurality of UV-LEDs (322) of the sterilizing module (320) into the interior of the package and/or by exposing the interior of the package for peroxide using a peroxide exposing unit (324) of the sterilizing module (320).

10. The system (300) according to claim 9, further comprising a conveyor line (210) configured to transport opening devices (100) along a conveyance path, the sterilizing unit (220) is arranged along the conveyance path, the conveyor line (210) extending from a source (230) of opening devices (100) to the opening device applicator (310), the conveyor line (210) comprises guiding members extending in a transport direction of the opening devices (100) along the conveyance path, wherein the opening devices (100) are placed in a queue with overlapping attachment flanges (112) in the conveyor line (210).

11. The system (300) according to claim 9 or 10, wherein the sterilizing module (320) comprises a plurality of UV-LEDs configured to emit UV-light into the interior of the package.

12. A method for sterilizing opening devices (100), each opening device (100) comprising a spout (110) provided with an attachment flange (112) and a cap (120) arranged to interact with the spout (110), wherein the opening device (100) has an inner top surface (116) comprising a circular protruding feature (114), the method comprising:
sterilizing (S402) the inner top surface (116) comprising the circular protruding feature (114) of the opening devices (100) during transport of the opening devices (100) to an opening device applicator (310) configured to apply the opening devices (100) to packages configured to contain liquid food, wherein the sterilizing (S402) is performed using UV-light emitted by a plurality of UV-LEDs arranged as at least two rings of UV-LEDs having different diameters, wherein the sterilizing (S402) the inner top surface (116) comprising the circular protruding feature (114) of the opening devices (100) comprises aligning a diameter of one of the two rings of UV-LEDs with a diameter of the protruding feature (114);
applying (S404) the opening device (100) to the package at the opening device applicator (310) by placing the opening device (100) in a pre-cut hole of the package and attaching the attachment flange (112) onto an attachment section on an inside of the package; and
sterilizing (S406) an interior of the package as well as a bottom surface of the attachment flange (112) of the opening device (100) applied onto the package using an sterilizing module (320).

13. The method according to claim 12, wherein sterilizing (S406) an interior of the package as well as a bottom surface of the attachment flange (112) of the opening device (100) applied onto the package comprises UV-light treatment by emitting UV-light from a plurality of UV-LEDs (322) of the sterilizing module (320) into the interior of the package.

14. The method according to claim 12 or 13, wherein sterilizing (S406) an interior of the package as well as a bottom surface of the attachment flange (112) of the opening device (100) applied onto the package comprises peroxide treatment by exposing the interior of the package for peroxide using a peroxide exposing unit (324) of the sterilizing module (320).
